# EUROPÄISCHE PATENTANMELDUNG

(11) **EP 3 045 107 A1**
(43) Veröffentlichungstag der Anmeldung: **20.07.2016**
(21) Anmeldenummer: 15400048.3
(22) Anmeldetag: 19.10.2015
(51) Int. Cl.: A61B 5/00, A61B 5/021, A61B 5/022

(54) **VORRICHTUNG ZUR BLUTDRUCKMESSUNG**

(30) Priorität: 19.01.2015 DE 102015000558
(71) Anmelder: seca ag, 4153 Reinach BL 1 (CH)
(72) Erfinder: Vogel, Frederik, 22301 Hamburg (DE)
(74) Vertreter: Patentanwälte Klickow & Partner Partnerschaftsgesellschaft mbB

(57) **Zusammenfassung**

Die Erfindung betrifft eine Vorrichtung zur Blutdruckmessung, aufweisend eine Einrichtung zur Blutdruckmessung und wenigstens eine daran gekoppelte Messwerteaufnahmevorrichtung, wobei die Vorrichtung zur Blutdruckmessung wenigstens eine Baueinheit und wenigstens eine Bedienungseinheit sowie Kopplungsmittel zur Kopplung wenigstens einer Messwerteaufnahmevorrichtung aufweist, sodass die Reduzierung beidhändiger Arbeitsschritte des Bedienpersonals unterstützt ist.

## Beschreibung

Die Erfindung betrifft eine Vorrichtung zur Blutdruckmessung, aufweisend eine Einrichtung zur Blutdruckmessung und wenigstens daran gekoppelte Messwerteaufnahmevorrichtung.

Zur Messung von Körpereigenschaften und Körperfunktionen von Subjekten, z.B. menschlichen Personen oder Tieren, werden sowohl mobile als auch stationäre Messgeräte eingesetzt. Bei den zu messenden Eigenschaften oder Funktionen handelt es sich häufig um Bio- beziehungsweise Körperimpedanzen, Herzfunktionen, Nervenfunktionen oder Elektrokardiogrammen. Weitere Messgrößen in diesem Zusammenhang können Körpergewicht, Körperzusammensetzungen, geometrische Abmessungen, Fingerabdrücke, Körpertemperaturen an verschiedenen Stellen, Zusammensetzung des verwerteten Atemgases, Atemgasvolumina, Blutdruckwerte usw. sein.

Insbesondere der Blutdruck eines Subjektes ist ein wichtiger Indikator für den Gesundheitszustand. Im Rahmen von Krankheitsdiagnosen ist die Ermittlung des Blutdruckes daher seit vielen Jahren ein Standardmessverfahren, das neben diversen anderen Körpereigenschaften und Körperfunktionen durchgeführt wird und als einzelner Messwert als eine Messwertkette pro Zeiteinheit und, oder in Verbindung mit weiteren Messwerten die verschiedenen anderen Körpereigenschaften und Körperfunktionen betreffend zur Diagnose von Krankheiten oder Risikozuständen herangezogen wird.

Unter dem Blutdruck eines Subjektes versteht man grundsätzlich den Druck des Blutes in einem Blutgefäß, wobei der Druck technisch gesehen eine Flächenpressung als Resultat einer auf ein Flächenelement rechtwinkelig wirkenden Kraftkomponente darstellt. Der Blutdruck ist abhängig von Herzleistung und Gefäßwiderstand und wird in mmHg (mm Quecksilbersäule) gemessen. Aufgrund der Tatsache, dass das Herz als Pumporgan diskontinuierlich arbeitet ist der Blutdruck nicht mit einem einzigen Druckwert beschreibbar. Vielmehr müssen zwei Werte ermittelt werden: Der systolische (obere) Blutdruck der entsteht, wenn das Herz kontrahiert und das Blut in die Blutgefäße drückt und der diastolische (untere) Blutdruck der sich einstellt, wenn sich der Herzmuskel in der Dehnungsphase ist und sich mit Blut füllt.

Der Blutdruck kann basierend auf verschiedenen Blutgefäßen ermittelt werden: Arterien, Venen oder an Blutgefäßen mit besonderen Aufgabenstellungen wie zum Beispiel Schlagadern. Von besonderem Interesse sind Blutdruckwerte in Arterien, d.h. arterielle Blutdruckwerte, weil deren Ermittlung in besonders einfacher und risikoloser Weise durch indirekte Messverfahren möglich ist. Bei diesen indirekten Messverfahren erfolgt die Ermittlung des Blutdruckes von ausserhalb des Körpers des Subjektes, das deshalb auch als unblutiges oder nichtinvasives Messverfahren bezeichnet wird.

Die indirekte Blutdruckmessung wird üblicherweise durch stationäre oder mobile Messgeräte realisiert, die elektronisch oder mechanisch-manuell arbeiten. Die Messwerteaufnahmevorrichtung für die Blutdruckmessung besteht häufig aus einer. Manschette, die als Druckmanschette ausgeführt ist und den Kontakt zwischen der zugeordneten Messwerteverarbeitungs- und Auswertevorrichtung und dem Subjekt herstellt.

Bei der indirekten Blutdruckmessung kommen verschiedene Messprinzipe zur Anwendung. Allen Prinzipen ist gemeinsam, dass die als Manschette ausgebildete Messwerteaufnahmevorrichtung an einer Extremität des Subjektes festgelegt wird. Die Blutdruckmessmanschette ist entweder schlauchförmig oder als zu einem Schlauch formbares flächenförmiges Band mit einem Verschlussmechanisimus ausgebildet und verfügt wenigstens abschnittsweise über ein Druckkissen. Das Druckkissen ist durch einen mit einem Medium befüllbaren, elastischen Hohlraum realisiert, der durch die druckbeaufschlagte Befüllung den Querschnitt vergrößert und dadurch eine für die Blutdruckmessung erwünschte Abschnürung der Extremität in Bezug auf die Blutzirkulation bewirkt.

Ist die Blutdruckmessmanschette schlauchförmig ausgebildet, so ist es erforderlich, dass diese vor der Messung der Extremität, häufig ein Arm des Subjektes, übergestülpt wird. Dazu muss das Bedienpersonal beidhändig arbeiten und es ist Voraussetzung, dass die Extremität nicht beispielsweise durch Infusionsvorrichtungen oder andere kabelgebundene Messwerteaufnahmevorrichtungen belegt ist, welche das Überstülpen unmöglich machen oder zumindest erschweren.

Ist die Blutdruckmessmanschette als zu einem Schlauch formbares flächenförmiges Band mit einem Verschlussmechanismus ausgebildet, erfolgt die Festlegung an einer Extremität des Subjektes durch Umlegen und Verschließen. Auch dazu muss das Bedienpersonal beidhändig arbeiten.

Die Blutdruckmanschette als Messwerteaufnahmevorrichtung für die Blutdruckmessung muss unabhängig von ihrer Ausgestaltung in Schlauchform oder flächenförmigem Band in ihrer relativen Lage an der Extremität in Position gehalten werden, bis durch die druckbeaufschlagte Befüllung des elastischen Hohlraums der Hohlraumquerschnitt soweit vergrößert und dadurch der Manschettendurchmesser soweit verkleinert ist, dass eine Klemmwirkung an der Extremität eintritt. Das bedeutet, dass das Bedienpersonal innerhalb dieses Arbeitsschrittes sowohl die druckbeaufschlagte Hohlraumbefüllung als auch die Manschettenfixierung gleichzeitig realisieren muss. Aufgrund dessen ist auch dieser Schritt durch beidhändiges Arbeiten geprägt.

Um mit den bekannten Messgeräten die indirekte Blutdruckmessung zu realisieren, kann ein weiterer Arbeitsschritt des Bedienpersonals erforderlich sein, der beidhändiges Arbeiten erfordert. Je nach verwendetem Messprinzip und, oder Ausgestaltung der Messvorrichtung kann die zeitgleiche Positionierung einer weiteren Messwertaufnahmevorrichtung, beispielsweise eines Stethoskops und, oder die Bedienung der zugeordneten Messwerteverarbeitungs- und Auswertevorrichtung ein beidhändiges Arbeiten erfordern.

Die beidhändigen Arbeitsschritte des Bedienpersonals bei der Blutdruckmessung stellen erhöhte Koordinationsanforderung dar und können Ursache für Fehlbedienungen und fehlerhaften Messungen sein. Darüber hinaus ist es dem Bedienpersonal in der Phase beidhändigen Arbeitens unmöglich, weitere Aufgaben neben der Blutdruckmessung durchzuführen.

Es ist daher Aufgabe der Erfindung, eine Vorrichtung zur Blutdruckmessung bereitzustellen, die beidhändige Arbeitsschritte des Bedienpersonals bei und für Blutdruckmessungen wenigstens reduziert.

Diese Aufgabenstellung löst die Erfindung durch eine Vorrichtung zur Blutdruckmessung, aufweisend wenigstens eine Einrichtung zur Blutdruckmessung, umfassend eine Bedienungseinheit sowie wenigstens eine zugeordneten Messwerteverarbeitungs- und Auswertevorrichtung, wobei die wenigstens eine Bedienungseinheit innerhalb einer Baueinheit angeordnet ist und die Baueinheit derart konstruktiv gestaltet ist, dass eine Einhandbedienung unterstützt wird und wenigstens eine Messwerteaufnahmevorrichtung an die Baueinheit koppelbar ist. Die erfindungsgemäße Lehre erkennt, dass sich beidhändige Arbeitsschritte des Bedienpersonals dadurch wenigstens reduzieren lassen, dass das Positionieren und Festlegen der Messwertaufnahmevorrichtung an einer Extremität des Subjektes und, oder die Bedienung der Vorrichtung zur Blutdruckmessung durch die geeignete haptische Gestaltung der Vorrichtung und vorrichtungstechnische Zusatzfunktionen erreichen lässt.

Die erfindungsgemäße Vorrichtung zur Blutdruckmessung des Systems ist konstruktiv primär durch die Baueinheit mit ihrer Möglichkeit zur Kopplung einer Messwerteaufnahmevorrichtung geprägt. Die Baueinheit weist wenigstens eine Bedienungseinheit auf und umfasst Kopplungsmittel zur Festlegung der wenigstens einen Messwerteaufnahmevorrichtung. Die Festlegung erfolgt vorzugsweise lösbar, sodass verschiedene Messwerteaufnahmevorrichtungen, beispielsweise für verschieden große Extremitäten, koppelbar sind.

Die Kopplungsmittel können innerhalb eines zur Baueinheit separaten Gehäuses angeordnet oder integraler Bestandteil der Baueinheit sein. Im Fall der Unterbringung der Kopplungsmittel in einem separaten Gehäuse kann die lösbare Verbindung der wenigstens einen Messwerteaufnahmevorrichtung dadurch realisiert sein, dass die Kopplungsmittel zusammen mit der Messwerteaufnahmevorrichtung von der Baueinheit lösbar sind.

Eine Ausführung der Vorrichtung zur Blutdruckmessung sieht einen Betätigungshebel vor, der mit dem Kopplungsmittel und, oder der Messwerteaufnahmevorrichtung korrespondiert. Die Baueinheit ist durch sein Gehäuse und die Anordnung der Bedieneinheit sowie des optionalen Betätigungshebels derart haptisch gestaltet, dass das Bedienpersonal die Vorrichtung zur Blutdruckmessung einhändig haltern und ohne umzugreifen sowohl die Bedieneinheit als auch den Betätigungshebel vorzugsweise durch wenigstens einen Finger der haltenden Hand bedienen kann.

Die Messwerteaufnahmevorrichtung umfasst wenigstens die für die indirekte Blutdruckmessung erforderlichen Mittel zur Lagefixierung und Klemmwirkung beziehungsweise Abschnürung der Extremität des Subjektes. Die Erfindung erkennt, dass die Reduzierung beidhändiger Arbeitsschritte des Bedienpersonals bei und für Blutdruckmessungen durch einen zangenartigen Aufbau der Messwerteaufnahmevorrichtung erreicht wird. Die Messwerteaufnahmevorrichtung besteht vorzugs- aber nicht notwendigerweise aus zwei Elementen, von denen wenigstens ein Element bewegbar ist.

Korrespondierend zu der Extremität ist wenigstens eines der Elemente sichelförmig gestaltet, sodass ein Umfassen beziehungsweise Umschließen einer Extremität des Subjektes wenigstens in Teilen des Extremitätenumfangs unterstützt wird.

Die Bewegbarkeit wenigstens eines der Elemente ist derart vorgesehen, dass das Element eine erste und wenigstens eine zweite Position einnehmen und zwischen diesen Positionen verschwenkt werden kann. Vorzugsweise wird die Extremität des Subjektes in einer der Positionen umfasst beziehungsweise umschlossen und in der anderen Position freigegeben. Die Freigabe ist durch eine entsprechende Maulweite endseitig der sichelförmigen Elemente zueinander dadurch sichergestellt, dass das Maulweitenmaß mindestens der maximalen Querschnittsbreite des üblicherweise runden oder ellipsenähnlichen Extremitätenquerschnittes entspricht.

Um die Lagefixierung und/oder Klemmwirkung beziehungsweise Abschnürung der Extremität des Subjektes für die Blutdruckmessung zu erzielen, sind die Elemente der Messwerteaufnahmevorrichtung entweder derart dimensioniert und auf die Extremitätengröße geometrisch abgestimmt, dass in der Umfassungbeziehungsweise Umschließungsposition die Klemmwirkung beziehungsweise Abschnürung der Extremität des Subjektes erreicht wird, oder es kann innenseitig wenigstens abschnittsweise an einem oder beiden Elementen ein Druckkissen mit einem oder mehreren elastischen Hohlräumen vorgesehen sein, die durch die druckbeaufschlagte Befüllung ihren Hohlraumquerschnitt soweit vergrößern, dass eine Klemm- und, oder Abschnürungswirkung an der Extremität eintritt.

Durch die Konstruktion der Messwerteaufnahmevorrichtung mittels wenigstens zweier Elemente in einer zangenartige Gestaltung ist ein axiales Einfädeln zur Positionierung an der Extremität nicht erforderlich, es kann vielmehr die Kontaktherstellung von Messwerteaufnahmevorrichtung zur Extremität des Subjektes in einfacher und die Einhandbedienung unterstützender Weise durch eine Radialbewegung der Vorrichtung zur Blutdruckmessung relativ zur Extremität erfolgen.

Die Erfindung sieht eine Reduzierung beidhändiger Arbeitsschritte des Bedienpersonals bei und für Blutdruckmessungen auch dadurch vor, dass die Bewegung oder Verschwenkung des wenigstens einen Elementes der Messwerteaufnahmevorrichtung durch einen Betätigungshebel ausgelöst und, oder gesteuert wird.

Die Erfindung wird nachfolgend mit Bezug auf die Figuren erläutert. Im Einzelnen zeigen
Fig. 1 die perspektivische Ansicht einer Vorrichtung zur Blutdruckmessung (1) aufweisend eine zugeordnete Messwerteverarbeitungs- und Auswertevorrichtung (40) und eine mittels Kopplungsmitteln (30) daran gekoppelte, räumlich getrennte Messwerteaufnahmevorrichtung (20),
Fig. 2 das erfindungsgemäße Ausführungsbeispiel eines Systems zur Blutdruckmessung mit einer Vorrichtung zur Blutdruckmessung (1) und einer Messwerteaufnahmevorrichtung (20) bei der Anwendung,
Fig. 3 in einer Seitenansicht das erfindungsgemäße Ausführungsbeispiel eines Systems zur Blutdruckmessung mit einer Vorrichtung zur Blutdruckmessung (1) aufweisend wenigstens eine Baueinheit (10) und eine mittels Kopplungsmitteln (30) daran gekoppelte Messwerteaufnahmevorrichtung (20),
Fig. 4 zeigt in der Draufsicht das erfindungsgemäße Ausführungsbeispiel eines Systems zur Blutdruckmessung aus Fig. 3,
Fig. 5 zeigt in der Draufsicht das erfindungsgemäße Ausführungsbeispiel eines Systems zur Blutdruckmessung wie bereits in Fig. 4 illustriert, jedoch mit einer Messwerteaufnahmevorrichtung (20) in der geöffneten Stellung.

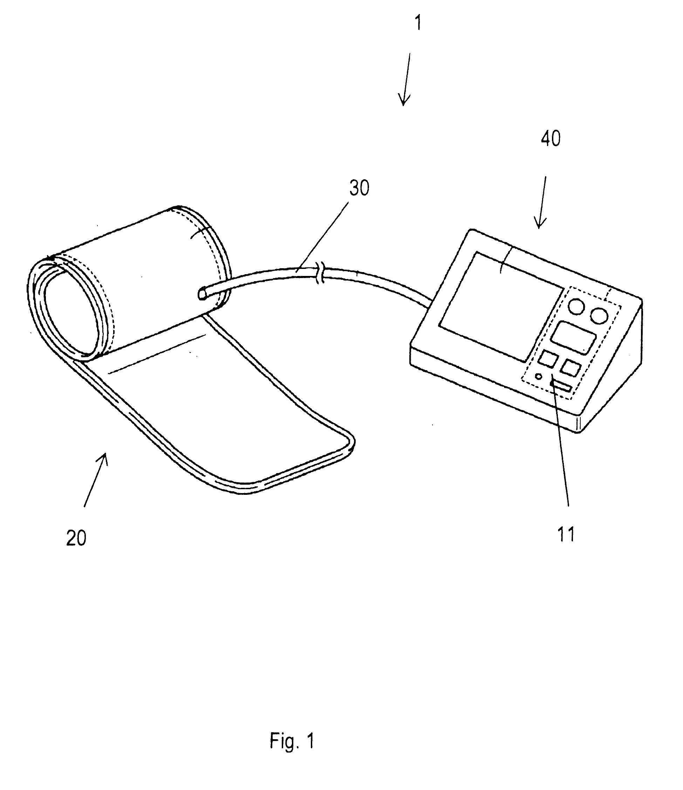

Figur 1 zeigt eine perspektivische Ansicht einer Vorrichtung zur Blutdruckmessung (1) aufweisend eine zugeordnete Messwerteverarbeitungs- und Auswertevorrichtung (40) und eine mittels Kopplungsmitteln (30) daran gekoppelte, räumlich getrennte Messwerteaufnahmevorrichtung (20) in Form einer Blutdruckmessmanschette, welche durch ein zu einem Schlauch formbares flächenförmiges Band mit einem Verschlussmechanismus ausgebildet ist.

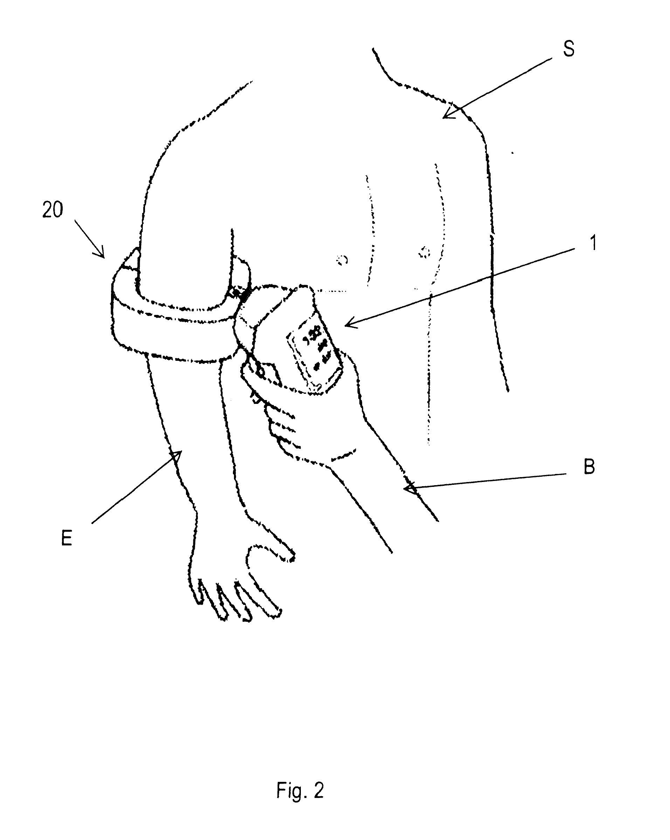

Figur 2 illustriert das erfindungsgemäße Ausführungsbeispiel eines Systems zur Blutdruckmessung mit einer Vorrichtung zur Blutdruckmessung (1) und einer Messwerteaufnahmevorrichtung (20) bei der Anwendung festgelegt an einer Extremität (E) eines Subjektes (S) und durch ein Bedienpersonal (B) bedient.

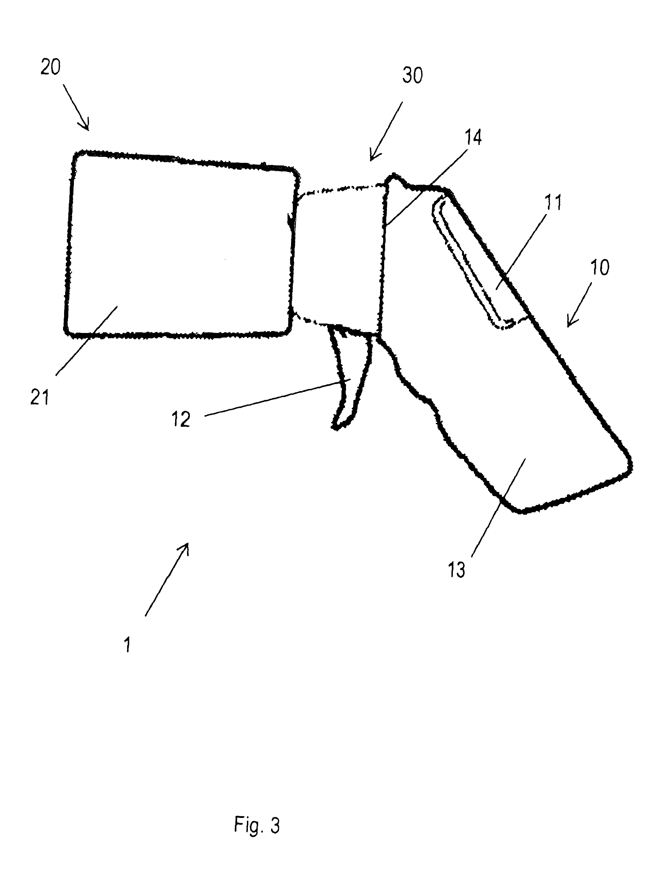

Figur 3 stellt in einer Seitenansicht das erfindungsgemäße Ausführungsbeispiel eines Systems zur Blutdruckmessung mit einer Vorrichtung zur Blutdruckmessung (1) aufweisend wenigstens eine Baueinheit (10) und eine mittels Kopplungsmitteln (30) daran gekoppelte Messwerteaufnahmevorrichtung (20) dar. Die Baueinheit (10) umfasst wenigstens ein Gehäuse in pistolengriffartiger Gestaltung und eine Bedienungseinheit (11). Um die Einhandbedienung durch das Bedienpersonal zu unterstützen ist die Baueinheit (10) haptisch derart konstruktiv gestaltet, dass die Vorrichtung zur Blutdruckmessung (1) mit einer Hand gehaltert werden kann. Die Bedienungseinheit (11) ist in dem Gehäuse der Baueinheit (10) vorzugsweise oberhalb einer Grifffläche (13) derart positioniert, sodass eine einhändige Bedienung unterstützt ist.

Die Bedienungseinheit (11) ist funktioneller, aber nicht notwendigerweise physischer Bestandteil der zugeordneten Messwerteverarbeitungs- und Auswertevorrichtung (40).

Die Erfindung sieht zwei mögliche Varianten vor: Entweder ist die zugeordnete Messwerteverarbeitungs- und Auswertevorrichtung (40) integraler Bestandteil der Baueinheit (10) und vorzugsweise innerhalb des Gehäuses der Baueinheit (10) angeordnet, oder die zugeordnete Messwerteverarbeitungs- und Auswertevorrichtung (40) ist als zu der Vorrichtung zur Blutdruckmessung (1) externes Gerät ausgebildet.

In dieser zweiten Variante ist die Vorrichtung zur Blutdruckmessung (1) praktisch ein Satellit, wobei eine Kommunikationsmöglichkeit zwischen der zugeordneten Messwerteverarbeitungs- und Auswertevorrichtung (40) und der Vorrichtung zur Blutdruckmessung (1) vorgesehen ist. Die Kommunikation kann durch Datenübertragung mittels kabelgebundener oder kabelloser Verfahren, gerätetechnisch realisiert durch Drahtlosübertragungseinrichtungen wie zum Beispiel Infrarot- oder WLAN- oder Bluetootheinrichtungen gebildet sein.

Weiterhin verfügt die Baueinheit (10) in dem Ausführungsbeispiel gemäß Figur 3 über eine Aufnahme (14) für das Anflanschen von Kopplungsmitteln (30), sodass eine Messwerteaufnahmevorrichtung (20) an die Baueinheit (10) koppelbar ist. Vorzugsweise erfolgt die Anflanschung durch eine lösbare kraft- und, oder formschlüssige Verbindung. Anstelle einer Aufnahme (14) kann das Kopplungsmittel (30) auch integraler Bestandteil innerhalb des Gehäuses der Baueinheit (10) sein.

Die Kopplung der Messwerteaufnahmevorrichtung (20) an der Baueinheit (10) durch die Kopplungsmittel (30) erfolgt derart, dass zum einen eine kompakte, einteilige Gerätschaft Kopplungsergebnis ist und zum anderen Steuerungs- und, oder Betätigungsfunktionen für die Messwerteaufnahmevorrichtung (20) von einem Betätigungshebel (12) und, oder von der Bedienungseinheit (11) über die Baueinheit (10) und durch die Kopplungsmittel (30) auf die Messwerteaufnahmevorrichtung (20) übertragbar sind. Der Betätigungshebel (12) kann entweder an dem Kopplungsmittel (30) oder an der Baueinheit (10) angeordnet sein.

Insbesondere umfassen die Steuerungsfunktionen beispielsweise die Aktivierung der druckbeaufschlagten Befüllung eines Hohlraums des wenigstens einen optionalen Druckkissens oder die Bewegung oder Verschwenkung des wenigstens einen Elementes (21) der Messwerteaufnahmevorrichtung (20).

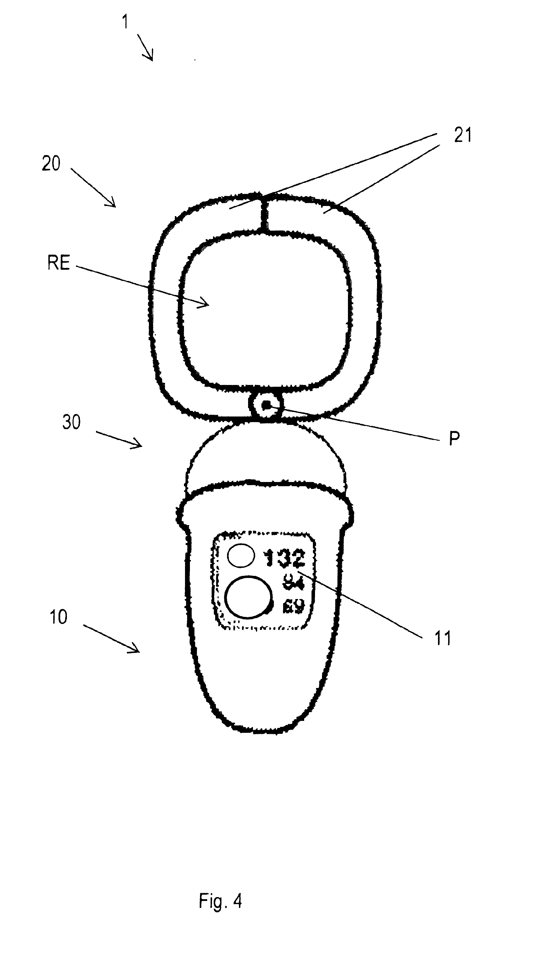

Figur 4 zeigt in der Draufsicht das erfindungsgemäße Ausführungsbeispiel eines Systems zur Blutdruckmessung mit einer Vorrichtung zur Blutdruckmessung (1), aufweisend wenigstens eine Baueinheit (10) und eine mittels Kopplungsmitteln (30) daran gekoppelte Messwerteaufnahmevorrichtung (20). In diesem Beispiel ist die Messwerteaufnahmevorrichtung (20) durch zwei sichelförmige Elemente (21) gebildet, die in einem Punkt (P) mit dem Kopplungsmittel (30) verbunden sind. Wenigstens eins der sichelförmigen Elemente (21) ist in Punkt (P) bewegbar und bilden zusammen eine zangenartige Anordnung die in ihrem Innern einen Extremitätenraum (RE) aufweist, sodass eine Extremität eines Subjektes umfasst werden kann. Die dargestellte Position der Elemente (21) der Messwerteaufnahmevorrichtung (20) entspricht der geschlossenen Position.

Abweichend von der in Figur 4 dargestellten achsensymmetrischen Anordnung von geometrisch weitgehend übereinstimmenden sichelförmigen Elementen (21) kann die Messwerteaufnahmevorrichtung (20) auch durch asymmetrisch angeordnete und, oder geometrisch von der Sichelform abweichende Elemente oder durch ein Element gebildet sein das geeignet ist, eine Extremität eine Subjektes zu umfassen.

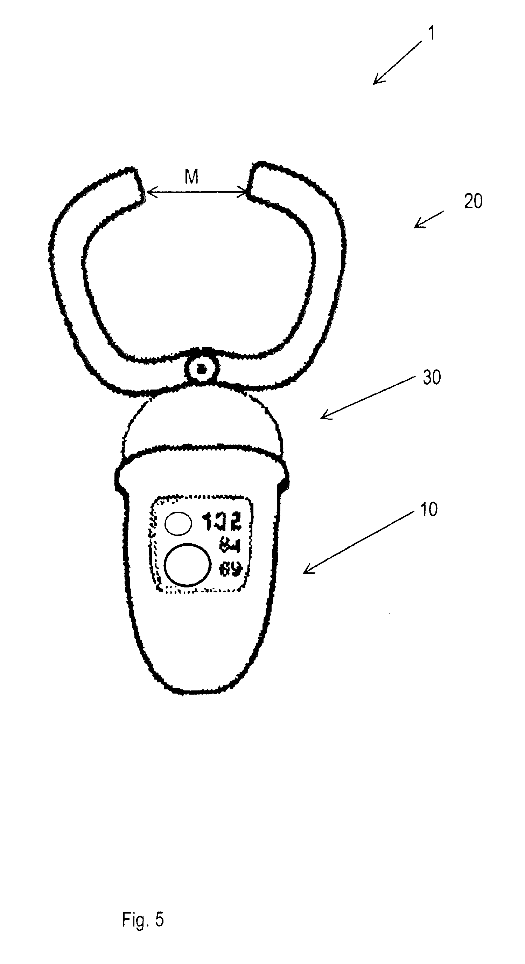

Figur 5 zeigt in der Draufsicht das erfindungsgemäße Ausführungsbeispiel eines Systems zur Blutdruckmessung mit einer Vorrichtung zur Blutdruckmessung (1) aufweisend wenigstens eine Baueinheit (10) und eine mittels Kopplungsmitteln (30) daran gekoppelte Messwerteaufnahmevorrichtung (20) wie bereits in Figur 4 illustriert, jedoch mit einer Messwerteaufnahmevorrichtung (20) in der geöffneten Stellung. Die Maulweite (M) entspricht mindestens der maximalen Querschnittsbreite des üblicherweise runden oder ellipsenähnlichen Extremitätenquerschnittes eines Subjektes.

Um die Bewegung des wenigstens einen Elementes (21) zu realisieren können, mechanische öder leistungsmotorische Wirkprinzip vorgesehen sein. Wird ein mechanisches Wirkprinzip gewählt ist insbesondere daran gedacht, durch ein innerhalb der Baueinheit (10) und, oder dem Kopplungsmittel (30) angeordnetes Hebelsystem die Bewegung auszulösen. Gesteuert wird das Hebelsystem durch den Betätigungshebel (12) oder die Bedienungseinheit (11).

Um bei mechanischen Wirkprinzipen die für die Bewegung des wenigstens einen Elementes (21) erforderlichen Antriebskräfte bereitzustellen, kann ausgehend von der geschlossenen Position durch Betätigung des Betätigungshebels (12) und der Aktivierung des Hebelsystems die Bewegung des wenigstens einen Elementes (21) in die geöffnete Stellung gegen eine Federkraft erfolgen. Wird der Betätigungshebel (12) freigegeben bewegt sich das wenigstens eine Element (21) federkraftgetrieben in die geschlossene Stellung.

Zusätzlich zu der Nutzung der Federkraft als Antriebskraft für die Bewegung von der geöffneten in die geschlossene Stellung des wenigstens einen Elementes (21) kann die Federvorspannung in der geschlossenen Stellung derart gewählt sein, dass eine definierte Schließkraft in der geschlossenen Position aufrecht erhalten wird, die die Klemmwirkung beziehungsweise Abschnürung der Extremität des Subjektes für die Blutdruckmessung unterstützt und, oder die Messwerteaufnahmevorrichtung (20) entgegen wenigstens eines optionalen, innenseitig eines Elementes (21) angebrachten Druckkissenkraft geschlossen hält.

Alternativ oder ergänzend zu der aus der Federkraft resultierenden Schließkraft kann die zuverlässige Geschlossenposition der Elemente (21) durch geeignete, lösbare form- oder kraftschlüssige Verbindungstechniken unterstützt werden. Denkbare Lösungen können Verrastungen, Hinterschneidungen, Haken- oder Greifzangenverbindungen, Magnetverschlüsse, selbsthemmende Keilverbindungen o.ä. sein, die durch die Betätigung des Betätigungshebels (12) oder der Bedienungseinheit (11) freigegeben werden und vorzugsweise endseitig der das Maul bildenden Elemente (21) angeordnet sind.

Weiterhin alternativ oder ergänzend zu den vorstehend beschriebenen Varianten kann zur Bereitstellung der geschlossenen Position auch bei Verrastungen und/oder magnetischen Verschlusskräften eine Einhandbedienung realisiert sein. Durch einen entsprechenden Betätigungsknopf oder einen Betätigungshebel kann beispielsweise eine Magnetkraft durch eine relativ zur bereitgestellten Haltekraft quer angerichtete Verschiebung mindestens eines der Magnete zumindest vermindert werden. Denkbar ist auch eine Kombination einer magnetischen Haltekraft durch eine elektromagnetisch erzeugte Gegenkraft.

Verrastungen können beispielsweise durch geeignete Hebelgestänge oder Seilzüge aufgehoben werden.

Insbesondere ist vorgesehen, dass die Messwerteaufnahmevorrichtung (20) lösbar mit dem Kopplungsmittel (30) verbunden ist.

Insbesondere ist vorgesehen, dass das Kopplungsmittel (30) ein an die Baueinheit (10) anfianschbares Bauelement ist und die Baueinheit (10) eine Aufnahme (14) zur Anflanschung des Kopplungsmittels (30) aufweist.

Insbesondere ist vorgesehen, dass die Aufnahme (14) der Baueinheit (10) eine Anflanschung des Kopplungsmittels (30) durch eine lösbare kraft- und, oder formschlüssige Verbindung unterstützt.

Insbesondere ist vorgesehen, dass das Kopplungsmittel (30) wenigstens ein Hebelsystem und eine Feder zur Bereitstellung einer Antriebskraft umfasst.

Insbesondere ist vorgesehen, dass die Feder des Kopplungsmittels (30) derart ausgebildet ist, das dessen Federvorspannung in der geschlossenen Stellung der Elemente (21) der Messwerteaufnahmevorrichtung (20) derart dimensioniert ist, dass eine definierte Schließkraft aufrecht erhalten wird, die die Klemmwirkung beziehungsweise Abschnürung der Extremität des Subjektes für die Blutdruckmessung unterstützt und, oder die Messwerteaufnahmevorrichtung (20) entgegen wenigstens eines optionalen, innenseitig eines Elementes (21) angebrachten Druckkissenkraft geschlossen hält.

Insbesondere ist vorgesehen, dass die Kopplung der Messwerteaufnahmevorrichtung (20) an der Baueinheit (10) durch das Kopplungsmittel (30) derart erfolgt, dass Steuerungs- und, oder Betätigungsfunktionen der koppelbaren Messwerteaufnahmevorrichtung (20) von einem Betätigungshebel (12) und, oder von der Bedienungseinheit (11) auf die koppelbare Messwerteaufnahmevorrichtung (20) übertragbar sind.

Insbesondere ist vorgesehen, dass eine Schließkraft durch mechanisch erzeugte Kräfte bereitgestellt wird.

Insbesondere ist vorgesehen, dass die Schließkraft durch mindestens einen Magneten und/oder mindestens eine Rastung bereitgestellt wird.

Insbesondere ist vorgesehen eine Vorrichtung zur Blutdruckmessung (1) nach einem der vorgehenden Ansprüche und wenigstens eine derart daran gekoppelte Messwerteaufnahmevorrichtung (20), dass ein kompaktes, einteiliges Blutdruckmesssystem Ergebnis dieser Kopplung ist, sodass die Reduzierung beidhändiger Arbeitsschritte des Bedienpersonals unterstützt ist.

Insbesondere ist vorgesehen, dass das System zur Blutdruckmessung mobil ist.

## Patentansprüche

1. Vorrichtung zur Blutdruckmessung (1), aufweisend wenigstens eine Baueinheit (10) und wenigstens eine Bedienungseinheit (11) sowie Kopplungsmittel (30) zur Kopplung wenigstens einer Messwerteaufnahmevorrichtung (20), **dadurch gekennzeichnet, dass** die Bedienungseinheit (11) in die Baueinheit (10) integriert und benachbart einer Grifffläche (13) positioniert ist, sodass eine einhändige Handhabung, Bedienung unterstützt ist.

2. Vorrichtung zur Blutdruckmessung (1) nach Anspruch 1, **gekennzeichnet dadurch, dass** die Bedienungseinheit (11) funktioneller Bestandteil einer Messwerteverarbeitungs- und Auswertevorrichtung (40) ist.

3. Vorrichtung zur Blutdruckmessung (1) nach Anspruch 1, **gekennzeichnet dadurch, dass** die Bedienungseinheit (11) integraler Bestandteil einer Messwerteverarbeitungs- und Auswertevorrichtung (40) ist, wobei die Messwerteverarbeitungs- und Auswertevorrichtung (40) innerhalb der Baueinheit (10) angeordnet ist.

4. Vorrichtung zur Blutdruckmessung (1) nach Anspruch 1, **gekennzeichnet dadurch, dass** die Baueinheit (10) ein Gehäuse in pistolengriffartiger Gestaltung umfasst und haptisch derart konstruktiv gestaltet ist, dass die Vorrichtung zur Blutdruckmessung (1) mit einer Hand gehaltert werden kann.

5. Vorrichtung zur Blutdruckmessung (1) nach Anspruch 1, **gekennzeichnet dadurch, dass** die Bedienungseinheit (11) in dem Gehäuse der Baueinheit (10) oberhalb einer Grifffläche (13) haptisch derart positioniert ist, sodass eine einhändige Bedienung durch das Bedienpersonal unterstützt ist.

6. Vorrichtung zur Blutdruckmessung (1) nach Anspruch 1, **gekennzeichnet dadurch, dass** ein Betätigungshebel (12) vorgesehen ist, durch den Steuerungs- und, oder Betätigungsfunktionen auf eine an die Vorrichtung zur Blutdruckmessung (1) koppelbare Messwerteaufnahmevorrichtung (20) ausübbar sind.

7. Vorrichtung zur Blutdruckmessung (1) nach Anspruch 6, **gekennzeichnet dadurch, dass** der Betätigungshebel (12) basierend auf mechanischen und, oder leistungsmotorischen Wirkprinzipen Steuerungs- und, oder Betätigungsfunktionen ermöglicht.

8. Vorrichtung zur Blutdruckmessung (1) nach Anspruch 6, **gekennzeichnet dadurch, dass** der Betätigungshebel (12) an dem Kopplungsmittel (30) oder an der Baueinheit (10) angeordnet ist.

9. Vorrichtung zur Blutdruckmessung (1) nach Anspruch 1, **gekennzeichnet dadurch, dass** die an die Baueinheit (10) koppelbare Messwerteaufnahmevorrichtung (20) wenigstens ein Element (21) aufweist.

10. Vorrichtung zur Blutdruckmessung (1) nach Anspruch 9, **gekennzeichnet dadurch, dass** die an die Baueinheit (10) koppelbare Messwerteaufnahmevorrichtung (20) durch zwei sichelförmige Elemente (21) gebildet ist, die in einem Punkt (P) mit dem Kopplungsmittel (30) verbunden sind.

11. Vorrichtung zur Blutdruckmessung (1) nach Anspruch 10, **gekennzeichnet dadurch, dass** die zwei sichelförmige Elemente (21) der an die Baueinheit (10) koppelbareri Messwerteaufnahmevorrichtung (20) eine zangenartige Anordnung bilden, die in ihrem Innern einen Extremitätenraum (RE) aufweist, sodass eine Extremität eines Subjektes umfassbar ist.

12. Vorrichtung zur Blutdruckmessung (1) nach Anspruch 10, **gekennzeichnet dadurch, dass** wenigstens eine der sichelförmigen Elemente (21) der an die Baueinheit (10) koppelbaren Messwerteaufnahmevorrichtung (20) in Punkt (P) zwischen einer offenen und einer geschlossenen Position bewegbar ist.

13. Vorrichtung zur Blutdruckmessung (1) nach Anspruch 10, **gekennzeichnet dadurch, dass** die Elemente (21) der an die Baueinheit (10) koppelbaren Messwerteaufnahmevorrichtung (20) in der offenen Stellung eine Maulweite (M) freigeben, die mindestens der maximalen Querschnittsbreite des üblicherweise runden oder ellipsenähnlichen Extremitätenquerschnittes eines Subjektes entspricht.

14. Vorrichtung zur Blutdruckmessung (1) nach Anspruch 9, **gekennzeichnet dadurch, dass** innenseitig wenigstens eines Elementes (21) der an die Baueinheit (10) koppelbaren Messwerteaufnahmevorrichtung (20) ein Druckkissen angebracht ist, die die Klemmwirkung beziehungsweise Abschnürung der Extremität des Subjektes für die Blutdruckmessung unterstützt.

15. Vorrichtung zur Blutdruckmessung (1) nach Anspruch 1, **gekennzeichnet dadurch, dass** das Kopplungsmittel (30) integraler Bestandteil innerhalb des Gehäuses der Baueinheit (10) ist.
